# EUROPEAN PATENT SPECIFICATION

(11) **EP 1 695 731 B1**
(45) Date of publication and mention of the grant of the patent: **24.02.2016**
(21) Application number: 04807490.0
(22) Date of filing: 15.12.2004
(51) Int. Cl.: A61M 16/16

(54) **HUMIDIFYING DEVICE AND OXYGEN CONCENTRATING SYSTEM**
BEFEUCHTUNGSVORRICHTUNG UND SAUERSTOFFKONZENTRATIONSSYSTEM
DISPOSITIF D'HUMIDIFICATION ET SYSTÈME DE CONCENTRATION D'OXYGÈNE

(30) Priority: 15.12.2003 JP 2003416308; 24.12.2003 JP 2003426457
(43) Date of publication of application: 30.08.2006
(73) Proprietor: Teijin Pharma Limited, Chiyoda-ku, Tokyo 100-0013 (JP); Ube Industries, Ltd., Ube-shi, Yamaguchi 755-8633 (JP)
(72) Inventor: TAKEDA, Toshihiro, c/o TEIJIN PHARMA LIMITED, Tokyo 100-0011 (JP); NISHIHIRA, Morihiko, Ota-ku, Tokyo 146-0083 (JP); TANIHARA, Nozomu, c/o Ube Chemical Factory, Ube-shi, Yamaguchi 755-8633 (JP)
(74) Representative: Hoffmann Eitle
(86) International application number: PCT/JP2004/019133
(87) International publication number: WO 2005/056092

(56) References cited:
- WO-A1-03/018096
- JP-A- 1 201 006
- JP-A- 2 099 113
- JP-A- 8 141 087
- JP-A- 8 141 087
- JP-A- 8 290 043
- JP-A- 9 276 408
- JP-A- 56 020 467
- JP-A- 61 101 405

## Description

### TECHNICAL FIELD

The present invention relates to a humidifying device for supplying moisture contained in air to a dry gas, to humidify the gas, and an oxygen concentrating system using such a humidifying device.

### BACKGROUND ART

As a humidifying device for humidifying a dry medical gas, a humidifying device of a gas bubble type or a evaporation type, which uses liquid water, is disclosed in, for example, Japanese Unexamined Patent Publication (Kokai) 06-238002. However, such a humidifying device using liquid water has problems in that it is necessary to periodically refill water in a container, or long-time usage may allow bacteria to propagate in the container or may make the water for humidification dirty. Therefore, the humidifying device must be washed periodically.

On the other hand, Japanese Unexamined Patent Publications (Kokai) Nos. 5-049697 and 8-141087 disclose humidifying devices, of a membrane type, which humidify a medical gas using water-permeable membranes. A humidifying device of a membrane type solves the above-mentioned problems because it uses water contained in the air. However, the humidifying device as disclosed in the above publications uses a compressed air, which may make it difficult to control the humidity, and a problem may appear in that, in particular, when a flow rate of the medical gas to be humidified is low, the gas is excessively humidified.

Further, Japanese unexamined Patent Publications (Kokai) Nos. 2000-237317 and 2000-237318 disclose humidifying devices, of a membrane type, using air at atmospheric pressure. In the humidifying devices as disclosed in the above-mentioned publications, however, it is difficult to bring a sufficient amount of air into contact with a moisture-permeable membrane, resulting in a problem in that the humidifying performance of the devices varies in accordance with the environmental conditions such as.the humidity of the air in the room in which the humidifying device is installed.

A device according to the preamble of claim 1 is known from JP-A- 8 141 087.

### DISCLOSURE OF THE INVENTION

Accordingly, an object of the present invention is to solve the problems in the prior art described above, and to provide a humidifying device capable of humidifying a gas to be humidified and, in particular, a dry medical gas, to the same degree as the atmospheric air, without using liquid water.

To achieve the above-mentioned object, according to the present invention, there is provided a humidifying device for humidifying a gas, with the water vapor contained in air, comprising: a hollow fiber bundle formed by bundling a plurality of hollow fibers permeable by water vapor, the hollow fibers being orientated in a direction of a predetermined axis; a housing having a space for accommodating the hollow fiber bundle therein, and having an introduction port for the gas to be humidified, communicating to bores of the hollow fibers, a discharging port for the gas to be humidified, communicating to the bores of the hollow fibers, an air inlet communicating to a space in the housing external of the hollow fibers, and an air exit communicating to the space in the housing external of the hollow fibers; and blowing means arranged at the air inlet of the housing for introducing air into the housing, wherein a ratio between the sum of the cross-sectional areas of the hollow fibers taken along a plane perpendicular to the axis, and the cross-sectional area of an air passage, is set within a range from 0.1 to 0.7, the cross-sectional area of the air passage being obtained by subtracting the sum of the cross-sectional areas of the hollow fibers from the cross-sectional area of the space of the housing taken along a plane perpendicular to the axis.

Further, the humidifying device according to the present invention could be used to humidify the oxygen-concentrated gas produced by an oxygen concentrating system for a medical use, the system adsorbing nitrogen contained in the air and removing it therefrom to produce an oxygen-concentrated gas for a medical use, and comprising: an oxygen concentrating section of a pressure-swing adsorption type having a plurality of adsorption columns, the columns respectively accommodating adsorbents having a selective absorbability for nitrogen; a conduit for introducing the oxygen-concentrated gas produced in the oxygen concentrating section to a user; pressure-adjustment means disposed in the conduit for adjusting a pressure at an exit of the oxygen concentrating section to a constant value; and flow rate regulating means for regulating a flow rate of the oxygen-concentrated gas flowing through the conduit to a constant value.

### BRIEF DESCRIPTION OF THE DRAWINGS

Fig. 1 is a block diagram of an oxygen concentrating system for a medical use to which the present invention is applied;
Fig. 2 is a schematic cross-sectional view of a humidifying device according to a first embodiment of the present invention;
Fig. 3 illustrates a cross-section taken along a line III-III in Fig. 2;
Fig. 4 is a graph illustrating a result of experiments carried out by using the humidifying device shown in Figs. 2 and 3;
Fig. 5 is a schematic cross-sectional view of a humidifying device according to a second embodiment of the present invention; and
Fig. 6 illustrates a cross-section taken along a line VI-VI in Fig. 5.

### BEST MODE FOR CARRYING OUT THE INVENTION

Preferred embodiments of the present invention will be described with reference to the attached drawings.

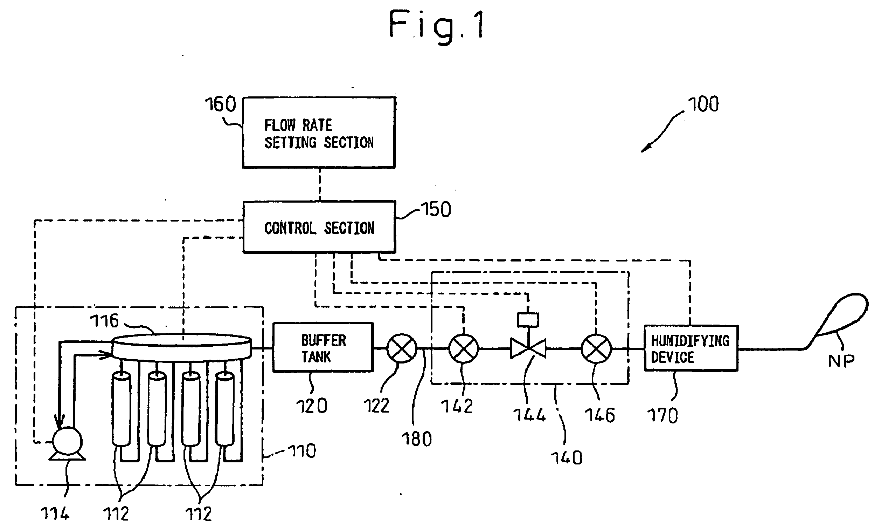

Referring to Fig. 1, an oxygen concentrating system for a medical use is illustrated as one example of a medical gas supplying system to which a humidifying device according to the present invention can be applied. The oxygen concentrating system 100 comprises an oxygen concentrating section 110 of a pressure-swing adsorption type, a buffer tank 120, a pressure-adjustment valve or a pressure-reducing valve 122 for regulating an exit pressure of the buffer tank 120 to a constant value, a flow rate regulating section 140, a flow rate setting section 160, a humidifying device 170, and a controlling section 150 for controlling the operation of the oxygen concentrating section 110, the flow rate regulating section 140 and the humidifying device 170. The oxygen-concentrated gas thus produced is supplied to a patient (not shown) via a pipe 180 and a nose cannula NP. The oxygen concentrating section 110 is an oxygen-concentrator of a four-column type provided with four adsorption columns 112, a compressor 114 capable of compression/decompression, and a rotary valve 116, and generates a gas containing approximately 90% absolutely dry oxygen.

The adsorption column 112 may be provided with a hollow tubular member formed of a material, such as metal, hardly permeable by gas and is filled therein with adsorbent having a selective absorbability to nitrogen. The adsorbent may be a molecular sieve of crystalline zeolite. Such zeolites are preferably those having metallic elements as cations and may include sodium zeolite X, lithium zeolite X or the like.

The flow rate regulating section 140 comprises a flow rate sensor 142 of a supersonic type, an automatic throttle valve means 144, and a pressure sensor 146 forming a respiration phase detecting means. The automatic throttle valve 144 comprises a linear valve of a solenoid type having a maximum orifice diameter of 1.7 mm. The pressure sensor 146 uses a pressure sensor having a pressure measurement range of ±75Pa, and a start of the inspiration phase is determined at a point in which the output of the pressure sensor 146 changes from a positive pressure to a negative pressure.

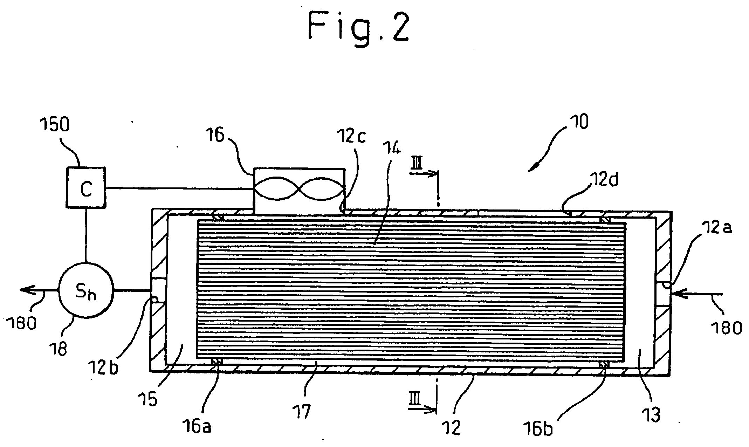

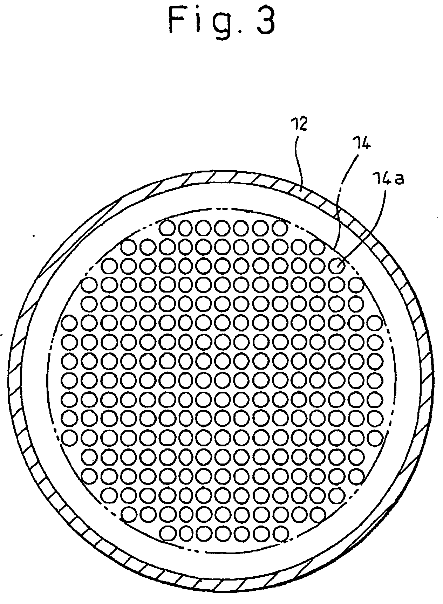

Next, a humidifying device according to a first embodiment of the present invention will be described with reference to Figs. 2 and 3.

The humidifying device 10 comprises a hollow circular-cylindrical housing 12 and a bundle 14 comprising a plurality of hollow fibers 14a disposed within the housing 12. Partition walls 16a and 16b are also provided in the housing 12, to divide the interior space of the housing 12 into an introduction chamber 15 for a gas to be humidified, which is located adjacent to an upstream end of the hollow fiber bundle 14 and communicates to the inner spaces of the hollow fibers 14a, a discharging chamber 15 for gas to be humidified, which is located adjacent to a downstream end of the hollow fiber bundle 14 and communicates to the inner spaces of the hollow fibers 14a, and an operation chamber 17 arranged between the introduction chamber 13 and the discharging chamber 15.

The housing 12 has an introduction port 12a for the gas to be humidified, which is formed in an upstream end wall and communicates to the introduction chamber 13, and an discharging port 12b for the gas to be humidified, which is formed in a downstream end wall and communicates to the discharging chamber 15. The introduction port 12a and the discharging port 12b are connected to the pipe 180. In a lateral wall of the housing 12, there are provided an air inlet 12c and an air exit 12d communicating to the operation chamber 17 which is an external space of the hollow fibers 14a, and a fan 16 is provided at the air inlet 12c for supplying the outside air into the operation chamber 17. A humidity sensor 18 is arranged in the pipe 180 downstream of the housing 12, and the control section 150 controls the rotational speed of the fan 16 to make the humidity detected by the humidity sensor 18 equal to a predetermined value.

The hollow fiber bundle 14 has 50 to 1,000 moisture permeable hollow fibers 14a. The respective hollow fiber 14a is preferably formed of fluoropolymer membrane having sulfonic acid as a functional group, such as Nafion membrane available from E. I. du Pont de Nemours & Company, a polyimide membrane or a polyether-imide membrane. Particularly, a polyimide membrane available from Ube Industries, Ltd. and a polyether-imide membrane available from Kuroda Precision Industries Ltd. have a respective water vapor permeation rate which hardly changes over time and, accordingly, are preferable. The number of the hollow fibers 14a is selected in accordance with a flow rate of the gas to be humidified, a target humidity, a water vapor permeation rate of the fiber 14a, a length and a diameter of the fiber 14a or an air flow rate of the fan 16, or the like.

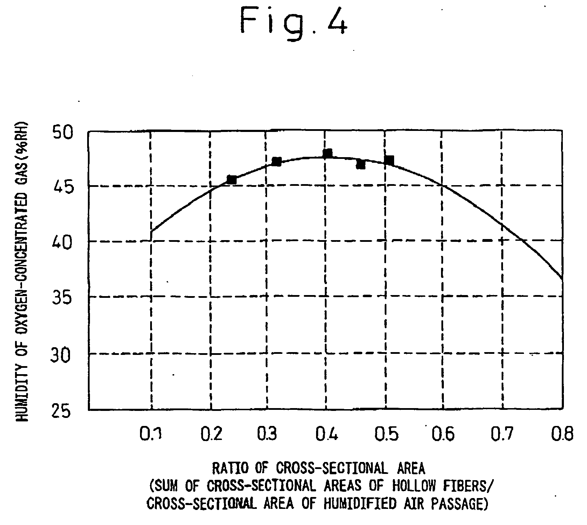

Fig. 4 is a graph obtained by experiments, carried out using the humidifying device shown in Figs. 2 and 3, illustrating the relationship of the humidity of the oxygen-concentrated gas, as a gas to be humidified, with respect to a ratio (the cross-sectional area ratio = ΣS_{hy}/Sₐₚ) between a sum of the cross-sectional areas of the hollow fibers 14a (ΣS_{hy}) and a cross-sectional area of an air passage. The cross-sectional area of an air passage is obtained by subtracting the sum of the cross-sectional areas of the hollow fibers 14a from the cross-sectional area Sₐₚ of the operation chamber 17. The experiments were carried out using the bundle 14 of 200 to 1,000 hollow fibers 14a formed of polyimide membrane, each having an inner diameter of approximately 400µm, an outer diameter of approximately 500µm, a length of 150 mm and a water vapor permeation rate of approximately 200×10⁻⁵ cm³ (STP)/(cm² sec cm Hg). As a gas to be humidified, oxygen-concentrated gas at 23°C was supplied at a flow rate of 5000 cm³/min. The fan 16 was a small-sized, axial flow fan of a low noise type, by which air of 23°C and 50%RH was supplied.

With reference to Fig. 4, when the cross-sectional area ratio is within a range from 0.1 to 0.7, the oxygen-concentrated gas in an approximately absolutely dry state is humidified to have a relative humidity of approximately 40%RH or more, and is usable in a medical gas supply system. Particularly, when the cross-sectional area ratio is within a range from 0.2 to 0.6, the oxygen-concentrated gas in an approximately absolutely dry state could be humidified to be the relative humidity of approximately 45%RH.

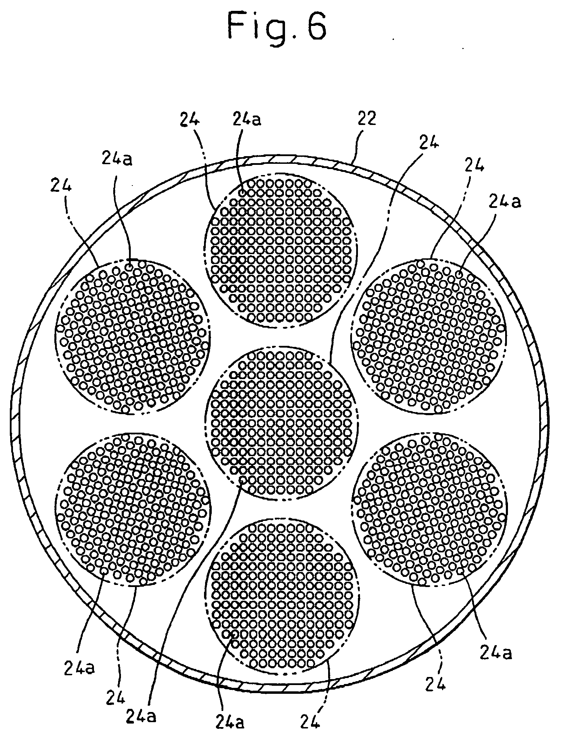

Next, the humidifying device according to a second embodiment of the present invention will be described with reference to Figs. 6 and 7.

The humidifying device 20 according to the second embodiment is structured generally in the same manner as in the humidifying device 10 according to the first embodiment, and comprises a hollow, circular-cylindrical housing 22 and a plurality of hollow fiber bundles 24 disposed in the housing 22, each of the plurality of hollow fiber bundles 24 comprising a plurality of hollow fibers 24a. In the interior of the housing 22, partition walls 16a and 16b are provided to divide the interior space of the housing 22 into an introduction chamber 23 for a gas to be humidified, which is located adjacent to an upstream end of the hollow fiber bundles 24 and communicates to the inner spaces of the hollow fibers 24a, a discharging chamber 25 for a gas to be humidified, which is located adjacent to a downstream end of the hollow fiber bundles 24 and communicates to the inner spaces of the hollow fibers 24a, and an operation chamber 27 arranged between the introduction chamber 23 and the discharging chamber 25.

The housing 22 has an introduction port 22a for the gas to be humidified, which is formed in an upstream end wall and communicates to the introduction chamber 23, and an discharging port 22b for the gas to be humidified, which is formed in a downstream end wall and communicates to the discharging chamber 25. The introduction port 22a and the discharging port 22b are connected to the pipe 180. In a lateral wall of the housing 22, there are provided an air inlet 22c and an air exit 22d communicating to the operation chamber 27 which is an external space of the hollow fibers 24a, and a fan 26 is provided at the air inlet 22c for supplying the outside air into the operation chamber 27. A humidity sensor 28 is arranged in the pipe 180 downstream of the housing 22, and the control section 150 controls the rotational speed of the fan 16 to make the humidity detected by the humidity sensor 18 equal to a predetermined value.

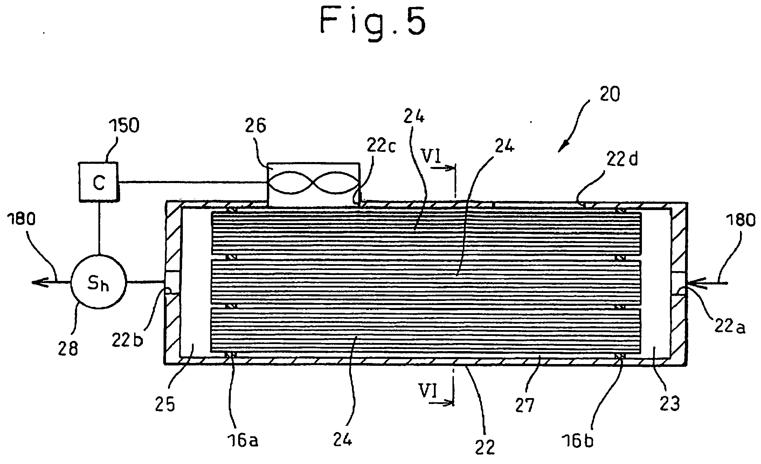

In the embodiment shown in Figs. 5 and 6, seven bundles 24 of the hollow fibers are provided, each bundle 24 has 100 hollow fibers 24a. The hollow fiber 24a may be of the same structure as that of the first hollow fiber 14a. The number of the hollow fibers 24a is selected in accordance with a flow rate of the gas to be humidified, a target humidity, a water vapor permeation rate, a length and a diameter of the hollow fiber 24a or an air flow rate of the fan 26, or the like, and the number of the hollow fiber bundles 24 is depended on the number of hollow fibers 14a to be used. For example, when 500 hollow fibers 24a are necessary, each of the hollow fiber bundles 24 may include 50 to 100 hollow fibers 24a; when 1,000 hollow fibers are necessary, each of the hollow fiber bundle 24 may have 50 to 250 hollow fibers 24a; when 2,000 hollow fibers are necessary, each of the hollow fiber bundle 24 may have 100 to 500 hollow fibers 24a; and when 5,000 hollow fibers are necessary, each of the hollow fiber bundle 24 may have 200 to 1,000 hollow fibers 24a.

Also, if the hollow fiber bundles 24 are accommodated in the housing 22 with an excessively large gap between the adjacent hollow fiber bundles 24, a so-called short-path occurs wherein air supplied by the fan 26 does not enter the hollow fiber bundles 24 but is discharged from the housing 22, whereby the humidifying performance is significantly deteriorated. Accordingly, it is necessary that the hollow fiber bundles 24 are disposed in the housing 22 with a gap of several millimeter or less between adjacent bundles.

While the preferred embodiments of the present invention have been described above, it will be, of course, apparent by persons with ordinary skill in the art that the present invention should not be limited thereto but may include various changes and modifications thereto.

While the supply system of the oxygen-concentrated gas for the medical use is described as a representative medical gas supply system applied to the present invention, the present invention should not be limited thereto but may be used for humidifying other medical gases such as nitrous oxide gas. Also, the humidifying device of the present invention may be used not only for humidifying the oxygen-concentrated gas obtained by separating nitrogen from air as medical gas but may also for humidifying oxygen gas produced by vaporizing liquid oxygen.

## Claims

1. A humidifying device (10, 20) for humidifying a gas, with the water vapour contained in air, comprising:
a hollow fiber bundle (14, 24) formed by bundling a plurality of hollow fibers (14a, 24a) permeable by water vapour, the hollow fibers being orientated in a direction of a predetermined axis;
a housing (12, 22) having a space for accommodating the hollow fiber bundle (14, 24) therein, and having an introduction port (12a, 22a) for the gas to be humidified, communicating to bores of the hollow fibers (14a, 24a), a discharging port (12b, 22b) for the gas to be humidified, communicating to the bores of the hollow fibers (14a, 24a), an air inlet (12c, 22c) communicating to a space in the housing (12, 22) external of the hollow fibers (14a, 24a) to introduce the outside air, and an air exit (12d, 22d) communicating to the space in the housing (12, 22) external of the hollow fibers (14a, 24a); and
**characterized in that**
a fan (16, 26) is arranged at the air inlet of the housing (12, 22) for introducing the outside air into the housing (12, 22), and
a ratio between a sum of the cross-sectional areas of the hollow fibers (14a, 24a) taken along a plane perpendicular to the axis, and the cross-sectional area of an air passage, is set within a range from 0.1 to 0.7, the cross-sectional area of the air passage being obtained by subtracting the sum (ΣS_{hy}) of the cross-sectional areas of the hollow fibers (14a, 24a) from the cross-sectional area (Sₐₚ) of the space of the housing (12, 22) taken along a plane perpendicular to the axis.

2. A humidifying device (10, 20) as defined by claim 1, wherein the ratio of the cross-sectional areas is set within a range from 0.2 to 0.6.

3. A humidifying device (10, 20) as defined by claim 1, wherein the hollow fiber is comprised of a polyimide membrane or a polyether-imide membrane.

4. A humidifying device (10, 20) as defined by claim 1, further comprising:
a humidity sensor (18, 28) provided at the discharging port for detecting the humidity of the gas to be humidified; and
a control section (150) for controlling the fan (16, 26) to make the humidity of the gas to be humidified, as detected by the humidity sensor, equal to a predetermined value.

5. A humidifying device (10) as defined by claim 1, wherein the gas to be humidified is an oxygen-concentrated gas.

6. An oxygen concentrating system (100), the system adsorbing nitrogen contained in the air and removing it therefrom to produce an oxygen-concentrated gas for a medical use, and comprising:
an oxygen concentrating section (110) of a pressure-swing adsorption type having a plurality of adsorption columns, the columns respectively accommodating adsorbents having a selective absorbability for nitrogen;
a conduit (180) for introducing the oxygen-concentrated gas produced in the oxygen concentrating section to a user;
pressure-adjustment means (122) disposed in the conduit (180) for adjusting a pressure at an exit of the oxygen concentrating section (110) to a constant value;
flow rate regulating means (140) for regulating a flow rate of the oxygen-concentrated gas flowing through the conduit to a constant value; and
the humidifying device (10, 20) as defined by claim 1.

## Patentansprüche

1. Befeuchtungsvorrichtung (10, 20) zum Befeuchten eines Gases mit in Luft enthaltenem Wasserdampf, wobei die Vorrichtung aufweist:
ein Hohlfaserbündel (14, 24), das durch Bündeln einer Vielzahl von Hohlfasern (14a, 24a) ausgebildet ist, die für Wasserdampf durchlässig sind, wobei die Hohlfasern in einer Richtung einer vorbestimmten Achse ausgerichtet sind;
ein Gehäuse (12, 22), das in sich einen Raum zum Aufnehmen des Hohlfaserbündels (14, 24) aufweist und eine Einführöffnung (12a, 22a) für das zu befeuchtende Gas, die mit Löchern der Hohlfasern (14a, 24a) in Verbindung steht, eine Abgabeöffnung (12b, 22b) für das zu befeuchtende Gas, die mit den Löchern der Hohlfasern (14a, 24a) in Verbindung steht, einen Lufteinlass (12c, 22c), der mit einem Raum in dem Gehäuse (12, 22) außerhalb der Hohlfasern (14a, 24a) in Verbindung steht, um die Außenluft einzuführen, und einen Luftauslass (12d, 22d) aufweist, der mit dem Raum in dem Gehäuse (12, 22) außerhalb der Hohlfasern (14a, 24a) in Verbindung steht; und
**dadurch gekennzeichnet, dass**
ein Ventilator (16, 26) bei dem Lufteinlass des Gehäuses (12, 22) zum Einführen der Außenluft in das Gehäuse (12, 22) angeordnet ist, und
ein Verhältnis zwischen einer Summe der Querschnittsflächen der Hohlfasern (14a, 24a) entlang einer Ebene senkrecht zu der Achse und der Querschnittsfläche eines Luftdurchgangs auf einen Bereich von 0,1 bis 0,7 eingestellt ist, wobei die Querschnittsfläche des Luftdurchgangs durch Subtrahieren der Summe (ΣS_{hy}) der Querschnittsflächen der Hohlfasern (14a, 24a) von der Querschnittsfläche (Sₐₚ) des Raums von dem Gehäuse (12, 22) entlang einer Ebene senkrecht zu der Achse ermittelt wird.

2. Befeuchtungsvorrichtung (10, 20) nach Anspruch 1, bei der das Verhältnis der Querschnittsflächen in einem Bereich von 0,2 bis 0,6 festgelegt ist.

3. Befeuchtungsvorrichtung (10, 20) nach Anspruch 1, bei der die Hohlfaser aus einer Polyimidmembran oder einer Polyetherimidmembran aufgebaut ist.

4. Befeuchtungsvorrichtung (10, 20) nach Anspruch 1, ferner mit:
einem Feuchtigkeitssensor (18, 28), der bei der Abgabeöffnung zum Erfassen der Feuchtigkeit des zu befeuchtenden Gases bereitgestellt ist; und
einem Steuerungsabschnitt (150) zum Steuern des Ventilators (16, 26), um die Feuchtigkeit des zu befeuchtenden Gases, wie durch den Feuchtigkeitssensor erfasst, einem vorbestimmten Wert anzugleichen.

5. Befeuchtungsvorrichtung (10) nach Anspruch 1, bei der das zu befeuchtende Gas ein sauerstoffangereichertes Gas ist.

6. Sauerstoffanreicherungssystem (100), wobei das System in der Luft enthaltenen Stickstoff adsorbiert und von dieser entfernt, um ein sauerstoffangereichertes Gas für eine medizinische Verwendung herzustellen, wobei das System aufweist:
einen Druckwechselabsorptionssauerstoffanreicherungsabschnitt (110), der eine Vielzahl von Adsorptionssäulen aufweist, wobei die Säulen jeweils ein Adsorptionsmittel aufnehmen, das eine gezielte Adsorptionsfähigkeit für Stickstoff aufweist;
einen Kanal (180) zum Einführen des sauerstoffangereicherten Gases in einen Benutzer, das in dem Sauerstoffanreicherungsabschnitt hergestellt worden ist;
ein Druckeinstellmittel (122), das in dem Kanal (180) zum Einstellen eines Drucks bei einem Ausgang des Sauerstoffkonzentrationsabschnitts (110) auf einen konstanten Wert angeordnet ist;
ein Strömungsgeschwindigkeitsregulierungsmittel (140) zum Regeln einer Strömungsgeschwindigkeit des sauerstoffangereicherten Gases, das durch den Kanal strömt, auf einen konstanten Wert; und
die Befeuchtungsvorrichtung (10, 20) nach Anspruch 1.

## Revendications

1. Dispositif d'humidification (10, 20) pour humidifier un gaz, avec la vapeur d'eau contenue dans l'air, comprenant :
un faisceau de fibres creuses (14, 24) formé par groupement d'une pluralité de fibres creuses (14a, 24a) perméable à la vapeur d'eau, les fibres creuses étant orientées dans la direction d'un axe prédéterminé ;
un boîtier (12, 22) ayant un espace pour loger le faisceau de fibres creuses (14, 24) à l'intérieur de celui-ci, et ayant un orifice d'introduction (12a, 22a) pour le gaz à humidifier, communiquant avec les lumières des fibres creuses (14a, 24a), un orifice de décharge (12b, 22b) pour le gaz à humidifier, communiquant avec les lumières des fibres creuses (14a, 24a), une entrée d'air (12c, 22c) communiquant avec un espace dans le boîtier (12, 22) externe des fibres creuses (14a, 24a) pour introduire l'air extérieur, et une sortie d'air (12d, 22d) communiquant avec l'espace dans le boîtier (12, 22) externe des fibres creuses (14a, 24a) ; et **caractérisé en ce que**
un ventilateur (16, 26) est agencé au niveau de l'entrée d'air du boîtier (12, 22) pour introduire l'air extérieur dans le boîtier (12, 22), et
le rapport entre la somme des aires de section transversale des fibres creuses (14a, 24a) définies le long d'un plan perpendiculaire à l'axe, et l'aire de section transversale d'un passage d'air, est défini dans une plage de 0,1 à 0,7, l'aire de section transversale du passage d'air étant obtenue par soustraction de la somme (ΣS_{hy}) des aires de section transversale des fibres creuses (14a, 24a) à l'aire de section transversale (Sₐₚ) de l'espace du boîtier (12, 22) défini le long d'un plan perpendiculaire à l'axe.

2. Dispositif d'humidification (10, 20) tel que défini par la revendication 1, dans lequel le rapport des aires de section transversale est défini dans une plage de 0,2 à 0,6.

3. Dispositif d'humidification (10, 20) tel que défini par la revendication 1, dans lequel la fibre creuse est constituée d'une membrane de polyimide ou d'une membrane de polyéther-imide.

4. Dispositif d'humidification (10, 20) tel que défini par la revendication 1, comprenant en outre :
un capteur d'humidité (18, 28) disposé au niveau de l'orifice de décharge pour détecter l'humidité du gaz à humidifier; et
une section de commande (150) pour commander le ventilateur (16, 26) pour amener l'humidité du gaz à humidifier, telle que détectée par le capteur d'humidité, à être égale à une valeur prédéterminée.

5. Dispositif d'humidification (10) tel que défini par la revendication 1, dans lequel le gaz à humidifier est un gaz concentré en oxygène.

6. Système de concentration d'oxygène (100), le système adsorbant l'azote contenu dans l'air et l'éliminant de celui-ci pour produire un gaz concentré en oxygène pour une utilisation médicale, et comprenant :
une section de concentration d'oxygène (110) d'un type d'adsorption modulée en pression ayant une pluralité de colonnes d'adsorption, les colonnes contenant respectivement des adsorbants ayant un pouvoir adsorbant sélectif pour l'azote ;
un conduit (180) pour introduire le gaz concentré en oxygène produit dans la section de concentration d'oxygène dans un utilisateur ;
un moyen d'ajustement de pression (122) disposé dans le conduit (180) pour ajuster une pression à une sortie de la section de concentration d'oxygène (110) à une valeur constante ;
un moyen de régulation de débit (140) pour réguler un débit du gaz concentré en oxygène s'écoulant à travers le conduit à une valeur constante ; et
le dispositif d'humidification (10, 20) tel que défini par la revendication 1.
